# EUROPEAN PATENT SPECIFICATION

(11) **EP 3 905 922 B1**
(45) Date of publication and mention of the grant of the patent: **20.12.2023**
(21) Application number: 19842544.9
(22) Date of filing: 26.12.2019
(51) Int. Cl.: A45D 34/04, A45D 40/26

(54) **SYSTEM FOR METERING IN A ROLLER BALL APPLICATOR**
SYSTEM ZUM DOSIEREN IN EINEM ROLLKUGELAPPLIKATOR
SYSTÈME DE DOSAGE DANS UN APPLICATEUR À BILLE

(30) Priority: 31.12.2018 US 201816236930
(43) Date of publication of application: 10.11.2021
(73) Proprietor: L'OREAL, 75008 Paris (FR); Cheng, Wenzhen, Clark NJ 07066 (US)
(72) Inventor: SOETERS, Blake, Clark, New Jersey 07066 (US); HOUGH, Rodney, Clark, New Jersey (US); CHENG, Wenzhen, Clark, New Jersey 07066 (US)
(74) Representative: Cabinet Nony
(86) International application number: PCT/US2019/068582
(87) International publication number: WO 2020/142348

(56) References cited:
- WO-A1-2016/033202
- DE-U1-202013 004 144
- FR-A1- 2 836 345
- US-A1- 2015 360 014

## Description

### CROSS-REFERENCE TO RELATED APPLICATIONS

This application claims the benefit of priority from U.S. Patent Application No. 16/236,930, "SYSTEM FOR METERING IN A ROLLER BALL APPLICATOR" filed on December 31, 2018.

### BACKGROUND

The "background" description provided herein is for the purpose of generally presenting the context of the disclosure. Work of the presently named inventors, to the extent it is described in this background section, as well as aspects of the description which may not otherwise qualify as prior art at the time of filing, are neither expressly or impliedly admitted as prior art against the present invention.

Applying a liquid, oil, or higher viscosity solution to a user's skin via a rollerball device may have applications in the fields of cosmetics, dermatology, myriad skin therapies, etc. As described in U.S. PG Publication No 2015/0360014A, solution in an interior of a reservoir may be in contact with a side of a rollerball facing the reservoir. As the rollerball is rolled along the skin of a user, the solution in the reservoir is transferred via the rollerball rotation to an exterior side of the rollerball in contact with the user's skin. Thus, the device facilitates a consistent application of solution over an area. This is especially advantageous when solutions containing medicine are applied to the skin and application via a user's hands may absorb a portion of the medicated solution. This may result in insufficient application of the recommended applied dosage. Furthermore, a user may attempt to compensate for this effect and apply more than the recommended dosage, resulting in inconsistent or over dosage. Accordingly, systems of dispensing metered quantities of solution are desired.

### SUMMARY

The present invention relates to systems for regulating application of solutions to a user as defined by claims 1 and 10. Preferred features of the invention are set out in the dependent claims.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
FIG. 1 is a perspective view schematic of a capsule, according to an exemplary aspect of the present invention;
FIG. 2 is a cross-sectional view schematic of a capsule, according to an exemplary aspect of the present invention;
FIG. 3 is a cross-sectional view schematic of a capsule during displacement of a push rod, according to an exemplary aspect of the present invention; and
FIG. 4 is a cross-sectional view schematic of a capsule for higher viscosity solutions, according to an exemplary aspect not according to the present invention.

### DETAILED DESCRIPTION

The description set forth below in connection with the appended drawings is intended as a description of various aspects of the disclosed subject matter and is not necessarily intended to represent the only aspect(s). In certain instances, the description includes specific details for the purpose of providing an understanding of the disclosed subject matter. However, it will be apparent to those skilled in the art that aspects may be practiced without these specific details. In some instances, well-known structures and components may be shown in block diagram form in order to avoid obscuring the concepts of the disclosed subject matter.

Reference throughout the specification to "one aspect" or "an aspect" means that a particular feature, structure, characteristic, operation, or function described in connection with an aspect is included in at least one aspect of the disclosed subject matter. Thus, any appearance of the phrases "in one aspect" or "in an aspect" in the specification is not necessarily referring to the same aspect. Further, the particular features, structures, characteristics, operations, or functions may be combined in any suitable manner in one or more aspects. Further, it is intended that aspects of the disclosed subject matter can and do cover modifications and variations of the described aspects.

It must be noted that, as used in the specification and the appended claims, the singular forms "a," "an," and "the" include plural referents unless the context clearly dictates otherwise. That is, unless clearly specified otherwise, as used herein the words "a" and "an" and the like carry the meaning of "one or more." Additionally, it is to be understood that terms such as "upper," "lower," "front," "rear," "side," "interior," "exterior," and the like that may be used herein, merely describe points of reference and do not necessarily limit aspects of the disclosed subject matter to any particular orientation or configuration. Furthermore, terms such as "first," "second," "third," etc., merely identify one of a number of portions, components, points of reference, operations and/or functions as described herein, and likewise do not necessarily limit aspects of the disclosed subject matter to any particular configuration or orientation.

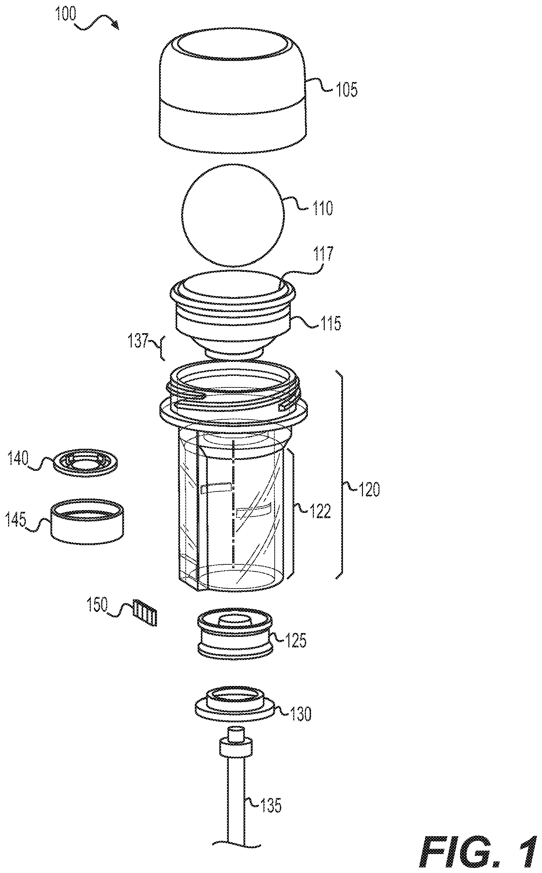

Fig. 1 illustrates a perspective view schematic of a capsule 100, according to an exemplary aspect of the present disclosure. The capsule 100 may include a cap 105, a rollerball 110, a fitment 115, a body 120, a piston 125, a plug 130, a push rod 135, and an authentication chip 150.

In one aspect, the cap 105 may be disposed at a first end of the body 120 and configured to attach to the body 120. For example, the cap 105 may be threaded and twist tightened onto the body 120 which may also be threaded (as shown) or the cap 105 may be snap tightened onto the body 120. The fitment 115 may be disposed at the first end of the body 120.

The cap 105, fitment 115, body 120, piston 125, and plug 130 may be fabricated from a polymer material. Non-limiting examples of materials for the cap 105, fitment 115, body 120, piston 125, and plug 130 (either separately or together) include at least one of a thermoplastic elastomer, polypropylene (PP), polyethylene terephthalate (PETG), acrylonitrile butadiene styrene (ABS), polycarbonate (PC), polyamide (Nylon), polystyrene (PS), low-density polyethylene (LDPE), and high-density polyethylene (HDPE), or any combination thereof. For example, all pieces may be fabricated from PP. In another example, the cap 105 may be fabricated from PP, the fitment 115 may be fabricated from PETG, the body 120 may be fabricated from PP, the piston 125 may be fabricated from LDPE, and the plug 130 may be fabricated from PP.

The body 120 may be shaped substantially cylindrical and include a first opening at the first end and a reservoir 122 at a second end, wherein an inner diameter of the first opening is wider than an inner diameter of the reservoir 122. Both the first opening and reservoir 122 may be substantially annular. The first opening of the body 120 may include a length of substantially straight stroke having the inner diameter of the first opening. The first opening may taper more narrowly down to the inner diameter of the reservoir 122. The reservoir 122 may be substantially straight and connected to the tapered portion extending from the first length of substantially straight stroke. It may be appreciated that the cross-sectional shape of the body 120 may be fabricated as myriad other shapes, for example triangular, square, pentagonal, hexagonal, octagonal, or the like.

The first opening may be configured to hold the fitment 115. The fitment 115 may include an exterior shape that is configured to be push-fit into the first opening, wherein the fitment 115 shape may contour to the length of substantially straight stroke and the tapered portion extending from the length of substantially straight stroke. Thus, the fitment 115 may form a liquid-tight seal with the first end of the body 120. In another aspect, the fitment 115 may be fabricated as part of the body 120 at the first end of the body 120. For example, the fitment 115 and body 120 may be molded together as one piece. The fitment 115 is configured to hold the rollerball 110, wherein an interior shape of the fitment 115 is substantially hemispherical. A first end of the fitment 115 may include a rollerball retainer 117. The rollerball retainer 117 may be an annular extrusion of material from the first end of the fitment 115 that may slightly taper inwards towards the interior of the fitment 115 such that the inner diameter of the rollerball retainer 117 is narrower than the diameter of the rollerball 110. The rollerball 110 may be installed in the fitment 115 by pushing the rollerball 110 through the opening of the rollerball retainer 117. The rollerball retainer 117 may elastically deform outwards (i.e. the rollerball retainer 117 opening widens and may thus be fabricated from a deformable polymer) to accommodate the rollerball 110 when the rollerball 110 is pushed through and then return to its original inner diameter. The rollerball 110 may be fabricated from glass, metal, or a polymer, such as the ones described for the cap 105, fitment 115, body 120, piston 125, and plug 130.

The piston 125 and plug 130 may be disposed at the second end of the body 120. The piston 125 may be shaped substantially disc-like and may include an outer diameter equal to, or marginally narrower than, the inner diameter of the reservoir 122 such that a liquid-tight seal may be formed between the piston 125 and an interior of the reservoir 122. The plug 130 may also be shaped substantially disc-like. The piston 125 may be installed in the reservoir 122 and the plug 130 may be installed at the second end of the body 120, wherein the plug 130 prevents egress of the piston 125. The plug 130 may be push-fit, snap-fit, twist-tightened, or chemically attached to the second end of the body 120. The plug 130 may include a hole in the middle configured to allow the push rod 135 to reversibly travel through. In another aspect, the plug 130 may be fabricated as a part of the body 120 at the second end of the body 120. For example, the plug 130 and body 120 may be molded together as one piece. It may be appreciated that the piston 125 and plug 130 may be shaped according to the cross-sectional shape of the body 120, and the disc-like shape is just one example.

A first end of the push rod 135 may be configured to abut the piston 125. For example, the piston 125 may include a molded indentation opening towards the second end of the body 120 having a shape complementary to the first end of the push rod 135. A second end of the push rod 135 (not shown) may be attached to a metering device (not shown) configured to translate the push rod 135 a predetermined distance. The metering device may take the form of the applicator described in the background, and as understood in the art, such an applicator would be configured to receive the capsule of the present embodiments. The abutting of the first end of the push rod 135 against the piston 125 therefore causes the piston 125 to travel towards the first end of the body (i.e. into the reservoir 122) the same predetermined distance the push rod 135 is translated.

The reservoir 122 is configured to hold a solution. In one aspect, the solution may be a cosmetic. Non-limiting examples include at least one of lip gloss, eye shadow, foundation, concealer, eyebrow liner, nail polish, and blush, or any combination thereof.

In one aspect, the solution may be a topical medication. Non-limiting examples include at least one of a serum, an ointment, a lotion, oil, an essential oil, a serum, a cream, a gel, a paste, foam, a water-based mixture, and an alcohol-based mixture (e.g. a tincture), or any combination thereof. The topical medication may include active ingredients, such as drug content, for treating skin ailments. In another aspect, the topical medication may include nutrients, for example vitamins and minerals.

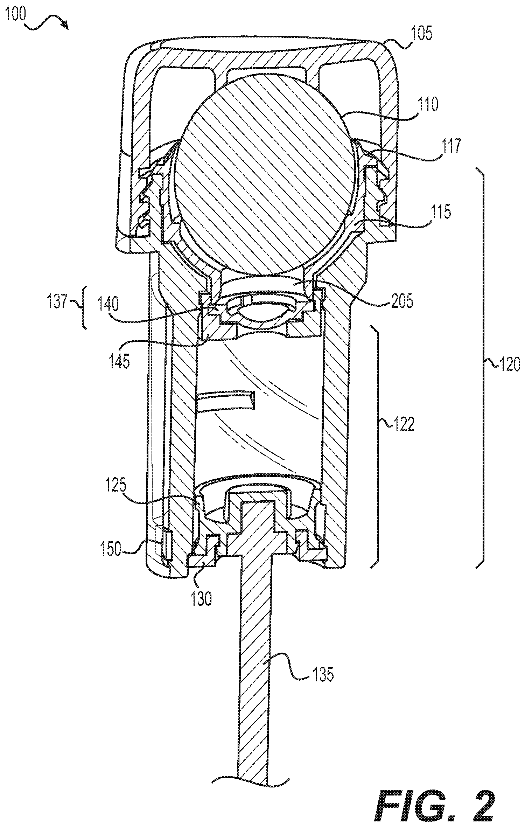

Fig. 2 illustrates a cross-sectional view schematic of the capsule 100, according to an exemplary aspect of the present disclosure. The fitment 115 includes a well 205. The 205 well may be the volume between the rollerball 110 and the interior of the fitment 115. The well 205 is configured to receive a predetermined volume of solution from the reservoir 122. The fitment 115 includes a solution regulator 137 disposed at a second end of the fitment 115 through which the predetermined volume of solution is transferred from the reservoir 122 to the well 205. The solution regulator 137 may be an orifice or a partially open orifice through which the solution flows towards the well 205, wherein the solution regulator 137 may be configured to meter the predetermined volume of solution passing through and preventing undesired reverse flow of solution from the well 205 towards the reservoir 122.

The solution regulator 137 is provided by a check valve 140. The solution regulator 137 may be substantially open and configured to allow attachment or insertion of the check valve 140. The check valve 140 may be installed inside or proximal to the second end of the fitment 115 and held in place via a check valve holder 145. The check valve 140 and check valve holder 145 may be installed in the reservoir 122 through the second end of the body 120. For example, the check valve 140 may be installed first and the check valve holder 145 may be installed after, wherein the check valve holder 145 includes features that allow it to be snap fit into complementary features of the reservoir 122. In another non-limiting example, the check valve 140 may be coupled to the check valve holder 145 prior to installation of both into the reservoir 122. In another non-limiting example, the check valve 140 and check valve holder 145 may be chemically bonded to the reservoir 122 by, for example, glue, epoxy, caulking, or any combination thereof. In another aspect, the check valve 140 and check valve holder 145 may be fabricated as a single part, i.e. the check valve 140 includes features that allow it to be snap fit into the complementary features of the reservoir without requiring the separate check valve holder 145. Non-limiting examples of materials for the check valve holder 145 include at least one of a thermoplastic elastomer, PP, PETG, ABS, PC, Nylon, PS, LDPE, and HDPE, or any combination thereof.

In one aspect, the check valve 140 may be a one-way valve allowing solution transfer in a single direction of flow (or preventing solution transfer in said direction of flow when flow stoppage is desired). The check valve 140 is a deformable membrane held in position via tension, wherein the position in tension forms a liquid-tight seal. For example, the check valve 140 may be fabricated from LDPE or PETG. In response to a force applied on the deformable membrane originating from a single direction, the membrane may deflect along the direction of the applied force. Upon release/ceasing of the applied force, the tension on the membrane may return the membrane to its un-deflected orientation. Thus, the check valve 140 may be in one of two states. As shown in Fig. 2, a first state may be closed and liquid-tight, wherein the check valve 140 does not allow solution from the reservoir 122 to transfer to the well 205. It may be appreciated by those in the art that other one-way valves may be used, for example a spring-ball construction.

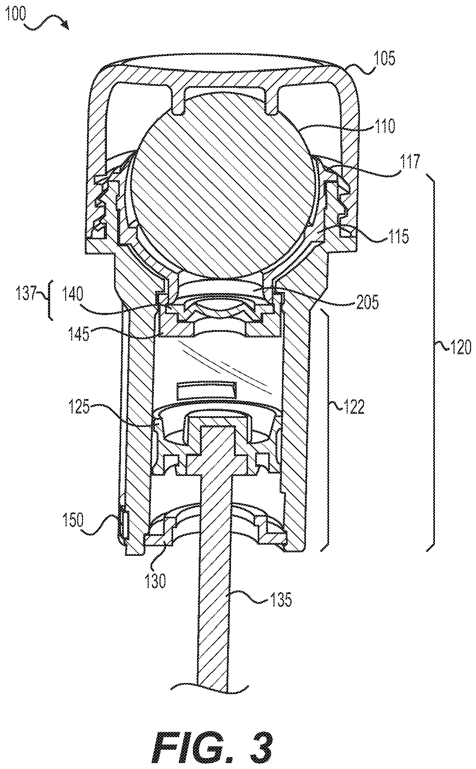

Fig. 3 illustrates a cross-sectional view schematic of the capsule 100 during displacement of the push rod 135, according to an exemplary aspect of the present disclosure. A second state of the check valve 140 may be open, wherein the check valve 140 membrane is deflected, thereby breaking the liquid-tight seal and allowing solution to transfer through the check valve 140.

In one aspect, the push rod 135 may be translated a predetermined distance. The push rod 135 may concomitantly translate the piston 125 the predetermined distance in the direction of the first end of the body 120. Since the solution in the reservoir 122 may not be compressible, the force of the piston 125 pushing on the solution may result in the check valve 140 switching from the first (closed) state to the second (open) state. The open check valve 140 may then allow the predetermined volume of solution to transfer from the reservoir 122 to the well 205. The rollerball 110 may be spherical and include a first portion of surface area in contact with the solution that was transferred to the well 205. The rollerball 110 may include a second portion of surface area exposed to the exterior and configured to contact a user's skin. The rollerball 110 may be configured to roll across the user's skin and transfer the predetermined volume of solution, for example the topical medication, from the well 205 to the user's skin. As the rollerball 110 is rotated over the user's skin and deposits the solution onto the user's skin, the second portion of surface area rolls into the well 205 and is coated again with more solution. Notably, the fitment 115 may be fabricated to include some play between the interior of the fitment 115 and the rollerball 110 to allow ease of rolling of the rollerball 110 and facilitate recoating of the rollerball 110 without the interior of the fitment 115 scraping off said coating of solution as the rollerball 110 rolls.

The predetermined distance the push rod 135 is translated may be determined by calculating the distance needed for the piston 125 to travel in order to displace the predetermined volume of solution in the reservoir 122. The maximum predetermined volume of solution transferred from the reservoir 122 may be determined by calculating the volume of solution the well 205 is capable of holding. The predetermined volume of solution actually transferred from the reservoir 122 to the well 205 may be determined by the metering device, for example the user may be attempting to complete a recommended regimen for treating a skin ailment. Thus, the user may desire a specific dosage of topical medication for applying to the user's skin and the metering device may be configured to transfer the predetermined volume of solution from the reservoir 122 to the well 205 at a predetermined frequency. For example, the metering device may transfer 0.3 mL of solution on a daily basis during a 14-day treatment plan, wherein the metering device is configured to allow the user to apply the solution within a preset length of time, for example 3 minutes per day. An on-board chip (not shown) in the metering device may record the user's usage and a position of the piston 125, wherein upon determining that the position of the piston 125 correlates to a 14^{th} day of the treatment, the metering device may notify the user to replace the capsule 100. In response to determining that the user has removed the capsule, the metering device may adjust and reset the position of the piston 125 to a position correlating to a start of the 14-day treatment plan. In addition, the metering device may reset the on-board chip to begin recording the user's usage again anew.

Advantageously, the built-in solution regulating feature, i.e. the check valve 140, may prevent excess solution from transferring to the well 205 once the piston 125 stops and the release of force (and the tension on the check valve 140) closes the check valve 140. Therefore, this prevents the user from over-applying said solution, which is especially important when the solution is a topical medication including a particular active ingredient, for example a drug, which should not be dosed in excess. Additionally, this may be aided by the metering device in which the capsule 100 is installed, wherein the metering device prevents the user from overdosing by only translating the piston 125 via the push rod 135 a predetermined number of instances within a predetermined timeframe, for example once per day, and not more frequently than programmed regardless of user input (e.g. the user prompting the metering device for another dose).

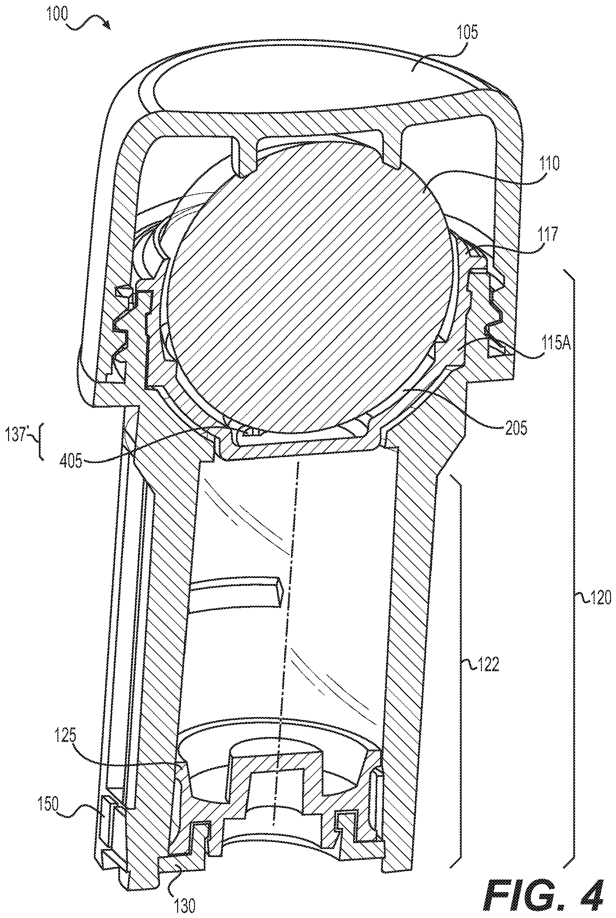

Fig. 4 illustrates a cross-sectional view schematic of the capsule 100 for higher viscosity solutions, not according to the present invention. In one aspect, a closed fitment 115a may be used including a first end and a second end like that of fitment 115. The closed fitment 115a may be fabricated similar to fitment 115 and configured for similar functionality, but fabricated such that the solution regulator 137' disposed at the second end of the fitment 115 is substantially closed and the flow metering for the solution regulator 137' is provided by at least one hole 405 (yielding closed fitment 115a). The at least one hole 405 may be disposed along the closed portion of the second end of the closed fitment 115a. In one aspect, the reservoir 122 may be filled with a high viscosity solution, such as ointment, gel, lotion, paste, or any solution having a viscosity sufficiently high enough to prevent the solution from moving through the at least one hole 405 without the translation of the piston 125. The size of the opening of the at least one hole 405 may be determined by the viscosity of the solution and a surface tension of the solution, wherein the size of the opening may be sufficiently narrow enough to prevent transfer of the solution through the at least one hole 405 due to a vacuum formed between the piston 125 and the interior of the reservoir 122, the viscosity of the solution providing friction forces to reduce flow of the solution, and the surface tension of the solution keeping the solution in the reservoir 122.

Notably, the capsule in this aspect includes a built-in passive solution regulating feature, i.e. the closed fitment 115a, that may prevent excess solution from transferring to the well 205 once the piston 125 stops. This again prevents the user from over-applying said solution, which is especially important when the solution is a topical medication including a particular active ingredient, for example a drug, which should not be dosed in excess.

It may be appreciated that the metering device may cause translation of the piston 125 via alternative methods. In an alternative aspect, the piston 125 may be magnetized via fabrication with magnetic materials or inclusion of magnets in or on the piston 125. For example, the piston 125 may be fabricated with polymers impregnated with magnetic metals. For example, a magnet may be attached to the surface of the piston 125. The metering device may include an electromagnet configured to attract or repel the magnetized piston 125. For example, the metering device electromagnet may repel the piston 125 the predetermined distance towards the first end of the body 120 in order to transfer the predetermined volume of solution from the reservoir 122 to the well 205.

In an alternative aspect, the piston 125 and push rod 135 may not be included and the capsule 100 sidewall may be thin and flexible. Instead of translating the push rod 135 the predetermined distance, the metering device may be configured to compress the flexible sidewall the predetermined volume to transfer the solution from the reservoir 122 to the well 205. The metering device may be configured to retain this compression until the user initiates a procedure to remove the capsule 100, wherein the metering device may release the compression to allow removal of the capsule 100.

In one aspect, the authentication chip 150 may be disposed on the surface of the body 120 and configured to communicate with the metering device in order to determine the authenticity of the capsule 100. For example, the metering device may read data on the authentication chip 150 and confirm that the capsule 100 was manufactured by an authorized retailer, such as L'Oréal or an approved L'Oréal subsidiary. The metering device may be configured to prevent usage of the capsule 100 if the authentication chip 150 does not confirm authenticity.

A number of implementations have been described. Nevertheless, it will be understood that various modifications may be made without departing from the scope of the invention as defined in the appended claims.

## Claims

1. A system (100) for regulating application of solution to a user, comprising:
a rollerball (110);
a body (120), including a first end and a reservoir (122) disposed at a second end of the body;
a fitment (115), including a well (205) and a solution regulator (137); and
a piston (125), wherein
the fitment is configured to hold the rollerball;
the rollerball is substantially spherical and rotatable in the fitment;
the first end of the body is configured to hold the fitment;
the reservoir is configured to hold a solution;
the piston is inserted into the reservoir at the second end of the body;
the well is configured to receive a predetermined volume of the solution from the reservoir; and **characterized in that**
the solution regulator includes a check valve attached to the fitment and disposed adjacent to the rollerball on an opposite side of the well, the solution regulator configured to allow transfer of the solution from the reservoir to the well in response to an applied force from a direction of the reservoir and prevent leakage of the solution from the well to the reservoir, the check valve including a deformable membrane held in a liquid-tight, closed state that deforms to an open state in response to the applied force.

2. The system of claim 1, wherein the solution is a topical medication.

3. The system of claim 1, wherein a volume of the well is defined by the space between an interior sidewalls of the fitment, the check valve attached to the fitment, and an exterior surface of the rollerball.

4. The system of claim 3, wherein the predetermined volume of the solution transferred through the solution regulator into the well is equal to or less than the volume of the well.

5. The system of claim 3, wherein the volume of the well is greater than 0.2 milliliters.

6. The system of claim 1, wherein the piston is configured to translate a predetermined distance towards the fitment and transfer the predetermined volume of the solution through the solution regulator into the well.

7. The system of claim 1, wherein the translation of the piston towards the fitment generates the applied force to open the check valve to allow transfer of the solution from the reservoir to the well.

8. The system of claim 1, further comprising:
a plug (130) disposed at the second end of the body and proximal to the piston on a side of the piston opposite the first end of the body; and
a push rod (135), wherein
the plug is attached to the body and configured to prevent egress of the piston through the second end of the body; and
the push rod abuts the piston and translating the piston a predetermined distance towards the fitment is performed by translating the push rod the predetermined distance towards the fitment.

9. The system of claim 10, further comprising:
an authentication chip configured to communicate with a metering device and provide authentication data.

10. A system (100) for regulating application of solution to a user, comprising:
a rollerball (110); and
a fitment (115), including a well (205) and a solution regulator (137), wherein
the fitment is configured to hold the rollerball;
the rollerball is substantially spherical and rotatable in the fitment;
the well is configured to receive a predetermined volume of the solution through the solution regulator; and **characterized in that**
the solution regulator includes a check valve attached to the fitment and disposed adjacent to the rollerball on an opposite side of the well, the solution regulator configured to allow transfer of the solution from a reservoir to the well in response to an applied force from a direction of the reservoir and prevent leakage of the solution from the well to the reservoir, the check valve including a deformable membrane held in a liquid-tight, closed state that deforms to an open state in response to the applied force.

11. The system of claim 1, wherein the fitment is removeably coupled to the first end of the body.

12. The system of claim 1, wherein the fitment is push-fit into the first end of the body.

13. The system of claim 10, wherein a volume of the well is defined by the space between interior sidewalls of the fitment, the check valve attached to the fitment, and an exterior surface of the rollerball.

## Patentansprüche

1. System (100) zum Regulieren des Auftragens einer Lösung auf einen Benutzer, umfassend:
eine Rollkugel (110),
einen Körper (120) mit einem ersten Ende und einem Behälter (122), der an einem zweiten Ende des Körpers angeordnet ist,
ein Passstück (115) mit einem Napf (205) und einem Lösungsregler (137) und
einen Kolben (125), wobei
das Passstück dazu ausgelegt ist, die Rollkugel zu halten,
die Rollkugel im Wesentlichen kugelförmig und in dem Passstück drehbar ist,
das erste Ende des Körpers dazu ausgelegt ist, das Passstück zu halten,
der Behälter dazu ausgelegt ist, eine Lösung zu enthalten,
der Kolben in den Behälter an dem zweiten Ende des Körpers eingesetzt ist,
der Napf dazu ausgelegt ist, eine vorbestimmte Menge der Lösung aus dem Behälter aufzunehmen, und **dadurch gekennzeichnet, dass**
der Lösungsregler ein Kontrollventil aufweist, das an dem Passstück angebracht und neben der Rollkugel auf einer gegenüberliegenden Seite des Napfes angeordnet ist, der Lösungsregler dazu ausgelegt ist, den Transfer der Lösung in Reaktion auf eine Krafteinwirkung aus einer Richtung des Behälters aus dem Behälter in den Napf zu ermöglichen und dazu, ein Austreten der Lösung aus dem Napf in den Behälter zu verhindern, wobei das Kontrollventil eine verformbare Membran aufweist, die in einem flüssigkeitsdichten, geschlossenen Zustand gehalten wird, der sich in Reaktion auf die aufgebrachte Kraft in einen offenen Zustand verformt.

2. System nach Anspruch 1, wobei die Lösung ein topisches Medikament ist.

3. System nach Anspruch 1, wobei ein Volumen des Napfes durch den Raum zwischen den inneren Seitenwänden des Passstücks, das an dem Passstück angebrachte Kontrollventil und eine Außenseite der Rollkugel definiert wird.

4. System nach Anspruch 3, wobei das vorbestimmte Volumen der durch den Lösungsregler in den Napf transferierten Lösung gleich dem oder geringer als das Volumen des Napfes ist.

5. System nach Anspruch 3, wobei das Volumen des Napfes größer ist als 0,2 Milliliter.

6. System nach Anspruch 1, wobei der Kolben dazu ausgelegt ist, einen vorbestimmten Abstand in Richtung des Passstücks zu translatieren und das vorbestimmte Volumen der Lösung durch den Lösungsregler in den Napf zu transferieren.

7. System nach Anspruch 1, wobei die Translationsbewegung des Kolbens in Richtung des Passstücks die Krafteinwirkung erzeugt, um das Kontrollventil zu öffnen, um den Transfer der Lösung aus dem Behälter in den Napf zu ermöglichen.

8. System nach Anspruch 1, ferner umfassend:
einen Stöpsel (130), der an dem zweiten Ende des Körpers und proximal zu dem Kolben auf einer Seite des Kolbens gegenüber dem ersten Ende des Körpers angeordnet ist, und
eine Schubstange (135), wobei
der Stöpsel an dem Körper angebracht und dazu ausgelegt ist, den Austritt des Kolbens durch das zweite Ende des Körpers zu verhindern, und
die Schubstange an den Kolben grenzt und das Translatieren des Kolbens eine vorbestimmte Distanz in Richtung des Passstücks durch Translatieren der Schubstange die vorbestimmte Distanz in Richtung des Passstücks durchgeführt wird.

9. System nach Anspruch 10, ferner umfassend:
einen Authentifizierungschip, der dazu ausgelegt ist, mit einer Dosiervorrichtung zu kommunizieren und Authentifizierungsdaten bereitzustellen.

10. System (100) zum Regulieren des Auftragens einer Lösung auf einen Benutzer, umfassend:
eine Rollkugel (110), und
ein Passstück (115) mit einem Napf (205) und einem Lösungsregler (137), wobei
das Passstück dazu ausgelegt ist, die Rollkugel zu halten,
die Rollkugel im Wesentlichen kugelförmig und in dem Passstück drehbar ist, der Napf dazu ausgelegt ist, ein vorbestimmtes Volumen der Lösung durch den Lösungsregler aufzunehmen, und **dadurch gekennzeichnet, dass**
der Lösungsregler ein Kontrollventil aufweist, das an dem Passstück angebracht und neben der Rollkugel auf einer gegenüberliegenden Seite des Napfes angeordnet ist, der Lösungsregler dazu ausgelegt ist, den Transfer der Lösung aus dem Behälter in den Napf in Reaktion auf eine Krafteinwirkung aus einer Richtung des Behälters zu ermöglichen und dazu, ein Austreten der Lösung aus dem Napf in den Behälter zu verhindern, wobei das Kontrollventil eine verformbare Membran aufweist, die in einem flüssigkeitsdichten, geschlossenen Zustand gehalten wird, der sich in Reaktion auf die Krafteinwirkung in einen offenen Zustand verformt.

11. System nach Anspruch 1, wobei das Passstück lösbar mit dem ersten Ende des Körpers gekoppelt ist.

12. System nach Anspruch 1, wobei das Passstück in das erste Ende des Körpers gesteckt ist.

13. System nach Anspruch 10, wobei ein Volumen des Napfes durch den Raum zwischen den Innenseitenwänden des Passstücks, das an dem Passstück angebrachte Kontrollventil und eine Außenseite der Rollkugel definiert wird.

## Revendications

1. Système (100) pour réguler l'application d'une solution à un utilisateur, comprenant :
une bille (110) ;
un corps (120), comprenant une première extrémité et un réservoir (122) disposé à une seconde extrémité du corps ;
un accessoire (115), comprenant un puits (205) et un régulateur de solution (137) ; et
un piston (125),
l'accessoire étant configuré pour contenir la bille ;
la bille étant sensiblement sphérique et rotative dans l'accessoire ;
la première extrémité du corps étant configurée pour maintenir l'accessoire ;
le réservoir étant configuré pour contenir une solution ;
le piston étant inséré dans le réservoir à la seconde extrémité du corps ;
le puits étant configuré pour recevoir un volume prédéterminé de la solution du réservoir ; et **caractérisé en ce que**
le régulateur de solution comprend un clapet anti-retour fixé à l'accessoire et disposé à côté de la bille sur un côté opposé du puits, le régulateur de solution étant configuré pour permettre le transfert de la solution du réservoir vers le puits en réponse à une force appliquée dans la direction du réservoir et pour empêcher la fuite de la solution du puits vers le réservoir, le clapet anti-retour comprenant une membrane déformable maintenue dans un état fermé, étanche aux liquides, qui se déforme à l'état ouvert en réponse à la force appliquée.

2. Système selon la revendication 1, la solution étant un médicament topique.

3. Système selon la revendication 1, un volume du puits étant défini par l'espace entre les parois intérieures de l'accessoire, le clapet anti-retour fixé à l'accessoire et la surface extérieure de la bille.

4. Système selon la revendication 3, le volume prédéterminé de la solution transférée à travers le régulateur de solution dans le puits étant égal ou inférieur au volume du puits.

5. Système selon la revendication 3, le volume du puits étant supérieur à 0,2 millilitre.

6. Système selon la revendication 1, le piston étant configuré pour se déplacer sur une distance prédéterminée vers l'accessoire et transférer le volume prédéterminé de la solution à travers le régulateur de solution dans le puits.

7. Système selon la revendication 1, la translation du piston vers l'accessoire générant la force appliquée pour ouvrir le clapet anti-retour afin de permettre le transfert de la solution du réservoir vers le puits.

8. Système selon la revendication 1, comprenant en outre :
un bouchon (130) disposé à la seconde extrémité du corps et proximal au piston sur un côté du piston opposé à la première extrémité du corps ; et
une tige de poussée (135),
le bouchon étant fixé au corps et configuré pour empêcher la sortie du piston par la seconde extrémité du corps ; et
la tige de poussée étant en butée contre le piston et la translation du piston sur une distance prédéterminée vers l'accessoire s'effectuant par la translation de la tige de poussée sur une distance prédéterminée vers l'accessoire.

9. Système selon la revendication 10, comprenant en outre :
une puce d'authentification configurée pour communiquer avec un dispositif de dosage et fournir des données d'authentification.

10. Système (100) permettant de réguler l'application d'une solution à un utilisateur, comprenant :
une bille (110) ; et
un accessoire (115) comprenant un puits (205) et un régulateur de solution (137), l'accessoire étant configuré pour maintenir la bille ;
la bille étant sensiblement sphérique et rotative dans l'accessoire ;
le puits étant configuré pour recevoir un volume prédéterminé de la solution à travers le régulateur de solution ; et **caractérisé en ce que**
le régulateur de solution comprend un clapet anti-retour fixé à l'accessoire et disposé à côté du ballon sur un côté opposé du puits, le régulateur de solution étant configuré pour permettre le transfert de la solution d'un réservoir au puits en réponse à une force appliquée dans la direction du réservoir et pour empêcher toute fuite de la solution du puits au réservoir, le clapet anti-retour comprenant une membrane déformable maintenue dans un état fermé, étanche aux liquides, qui se déforme à l'état ouvert en réponse à la force appliquée.

11. Système selon la revendication 1, l'accessoire étant couplé de manière amovible à la première extrémité du corps.

12. Système selon la revendication 1, l'accessoire étant emboîté dans la première extrémité du corps.

13. Système selon la revendication 10, un volume du puits étant défini par l'espace entre des parois intérieures de l'accessoire, le clapet anti-retour fixé à l'accessoire et une surface extérieure de la bille.
